# DEMANDE DE BREVET EUROPEEN

(11) **EP 1 090 623 A1**
(43) Date de publication de la demande: **11.04.2001**
(21) Numéro de dépôt: 00402663.9
(22) Date de dépôt: 26.09.2000
(51) Int. Cl.: A61K 7/06

(54) **Composition de lavage des matières kératiniques, à base d'un agent tensio-actif détergent, d'un agent nacrant et/ou opacifiant et d'un terpolymère acrylique**

(30) Priorité: 29.09.1999 FR 9912169
(71) Demandeur: L'OREAL, 75008 Paris (FR)
(72) Inventeur: Maurin, Véronique, 75016 Paris (FR); Beauquey, Bernard, 92110 Clichy (FR)
(74) Mandataire: Casalonga, Axel

(57) **Abrégé**

Composition de lavage des matières kératiniques comprenant dans un milieu cosmétiquement acceptable:
i) au moins un agent tensio-actif détergent anionique, amphotère ou non-ionique;
ii) au moins 1,5% en poids par rapport au poids total de la composition, un agent nacrant et/ou opacifiant; et
iii) au moins un terpolymère acrylique constitué d'un monomère (a) choisi parmi un acrylate d'alkyle en C₁-C₆ et un méthacrylate d'alkyle en C₁-C₆; d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di-(C₁-C₄)alkylamino (C₁-C₄) alkyl (méth)acrylamide; d'un monomère (c) choisi parmi un uréthane produit par réaction entre un isocyanate insaturé monoéthylénique et un agent tensioactif non ionique, un monomère tensioactif éthylénique copolymérisable, un monomère tensioactif de type urée, un éther d'allyle à groupements alkylèneoxy et un monomère non-ionique de type uréthane.

## Description

La présente invention concerne d'une manière générale des compositions de lavage des matières kératiniques, à base d'un agent tensio-actif détergent, d'un agent nacrant et/ou opacifiant et d'un terpolymère acrylique, ainsi qu'un procédé de lavage mettant en oeuvre ces compositions.

Les agents nacrants et/ou opacifiants sont couramment utilisés dans les shampooings afin de conférer à ces derniers un aspect nacré, préféré par le consommateur. Il a été constaté que ces agents nacrants, du fait de leur faible densité, présentaient souvent l'inconvénient de remonter à la surface du shampooing et d'y former une couche inesthétique pour le consommateur. Pour éviter l'apparition de ce phénomène, des polymères, notamment d'acide acrylique réticulé tels que les Carbopols, sont fréquemment utilisés; toutefois ils présentent l'inconvénient de diminuer les performances cosmétiques des shampooings, notamment en rendant les fibres kératiniques plus rêches et plus chargées.

Il existe donc un besoin d'une composition cosmétique détergente, en particulier un shampooing, qui présente un aspect nacré et/ou opacifié tout en permettant d'obtenir des performances cosmétiques acceptables au niveau des matières kératiniques, à savoir notamment les cheveux et le cuir chevelu; et plus particulièrement au niveau de la légèreté, de la douceur et du toucher des cheveux.

La demanderesse a découvert, de façon surprenante, qu'il était possible de formuler des compositions de lavage des matières kératiniques, notamment des shampooings présentant un aspect nacré et/ou opacifié tout en ayant les propriétés esthétiques et cosmétiques recherchées, en utilisant dans ces compositions un agent tensio-actif détergent et un agent nacrant et/ou opacifiant associés à un terpolymère acrylique spécifique, défini ci-après. En effet, il a été constaté que l'utilisation dudit terpolymère acrylique permettait d'obtenir sur les matières kératiniques, et notamment les cheveux des propriétés cosmétiques satisfaisantes, particulièrement en les rendant plus légers et en conférant aux cheveux mouillés un toucher doux et aux cheveux séchés, plus de douceur, de lissage, de souplesse, de malléabilité et de brillance.

Il a été également constaté que les compositions selon l'invention présentaient une bonne solubilité et une bonne tolérance cutanée.

L'invention a donc pour objet des compositions de lavage des matières kératiniques essentiellement caractérisées en ce qu'elles comprennent dans un milieu cosmétiquement acceptable:
1) au moins un agent tensio-actif détergent anionique, amphotère ou non-ionique;
2) au moins 1,5% en poids par rapport au poids total de la composition d'au moins un agent nacrant et/ou opacifiant choisi parmi :
   i) les esters de polyols à plus de deux atomes de carbone et d'acides gras à chaîne longue;
   ii) les alcanolamides d'acides gras à chaîne longue;
   iii) les esters de monoalcools à chaîne longue et d'acides gras à chaîne longue;
   iv) les éthers d'alcools gras à chaîne longue;
   v) les esters à chaîne longue d'alcanolamides à chaîne longue;
   vi) les alcools gras monochaînes à plus de 20 atomes de carbone;
   vii) les oxydes d'amines à chaîne longue;
   viii) l'acide benzoïque N,N-dihydrocarbyl(C₁₀-C₃₀, préférentiellement C₁₂-C₂₂) amido et leurs sels;
   ix) les alcools ayant de 27 à 48 atomes de carbone et comportant un ou deux groupements éther et/ou thioéther ou sulfoxyde; et
   x) les oxydes de titane et les micas;
3) et au moins un terpolymère acrylique constitué:
   - de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
   - de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di-(C₁-C₄)alkylamino (C₁-C₄) alkyl (méth)acrylamide;
   - de 0,1 à 30% en poids, préférentiellement de 0,1 à 10% en poids d'un monomère (c) choisi parmi :
      un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
      un monomère tensioactif insaturé éthylénique copolymérisable obtenu par condensation d'un tensioactif non-ionique avec un acide carboxylique insaturé α,β-éthylénique ou son anhydride;
      un monomère tensioactif choisi parmi les produits de réaction de type urée d'un monoisocyanate insaturé monoéthylénique avec un tensioactif non-ionique présentant une fonction amine;
      un éther de (méth) allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne l'éthylèneoxy, n est égal à zéro ou désigne un nombre entier inférieur ou égal à 200 et préférentiellement inférieur à 100, m et p désignent zéro ou un nombre entier inférieur à n et R₂ est un groupe hydrophobe d'au moins 8 atomes de carbone et préférentiellement en C₈-C₃₀; et un monomère non-ionique de type uréthane produit par réaction d'un tensioactif non ionique monohydrique avec un isocyanate insaturé monoéthylénique;
      les pourcentages en poids de monomères étant basés sur le poids total des monomères constituant le terpolymère.

Dans la composition de lavage selon l'invention, le terpolymère acrylique est présent à raison de 0,01 à 20 % , de préférence 0,1 à 10 % en poids de matière active (M.A.) par rapport au poids total de la composition.

Des monomères acrylates (a) préférés comprennent notamment les acrylates d'alkyle en C₂-C₆. L'acrylate d'éthyle est tout particulièrement préféré.

Comme exemples de monomères (b) préférés, il faut citer le méthacrylate de N,N-diméthylaminoéthyle (DMAEMA), l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyle-méthacrylamide, le N,N-diéthylaminopropylacrylamide et le N,N-diéthylaminopropyl-méthacrylamide. Le méthacrylate de N,N-diméthylaminoéthyle est tout particulièrement préféré.

Les monomères (c) préférés sont les monomères tensio-actifs insaturés éthyléniques copolymérisables obtenus par condensation d'un tensioactif non-ionique avec un acide carboxylique insaturé α,β-éthylénique ou son anhydride, de préférence les acides mono ou dicarboxyliques en C₃-C₄ ou leurs anhydrides et plus particulièrement l'acide acrylique, l'acide méthacrylique, l'acide crotonique, l'acide maléique, l'anhydride maléique et tout particulièrement l'acide itaconique et l'anhydride itaconique.

Les monomères (c) particulièrement préférés correspondent aux monomères tensioactifs insaturés éthyléniques copolymérisables obtenus par condensation d'un tensioactif non-ionique avec l'acide itaconique. Parmi les tensioactifs non-ioniques, on peut citer notamment les alcools gras en C₁₀-C₃₀ alkoxylés avec 2 à 100, et de préférence de 5 à 50 moles d'oxyde d'alkylène, comme par exemple les éthers de polyéthylène glycol et d'alcools gras en C₁₀-C₃₀ et plus particulièrement les éthers de polyéthylène glycol et d'alcool cétylique, dénommés CETETH dans le dictionnaire CTFA, 7ème édition, 1997.

Des méthodes conventionnelles pour préparer ces terpolymères acryliques sont connues de l'homme du métier. De telles méthodes incluent la polymérisation en solution, la polymérisation par précipitation et la polymérisation en émulsion par exemple. Des terpolymères conformes à l'invention et leurs méthodes de préparation sont notamment décrits dans les demandes EP-A-0824914 et EP-A-0825200.

Parmi ces terpolymères, on préfère utiliser en particulier le polymère "STRUCTURE ® PLUS" vendu par la Société NATIONAL STARCH, qui est constitué d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A..

En plus de ces monomères, le terpolymère peut contenir d'autres monomères qui permettent de réticuler ledit terpolymère. Ces monomères sont utilisés dans des proportions assez faibles, jusqu'à 2% en poids par rapport au poids total des monomères utilisés pour préparer le terpolymère. De tels monomères de réticulation comprennent des monomères aromatiques portant plusieurs substituants vinyle, des monomères alicycliques portant plusieurs substituants vinyle, des esters bi-fonctionnels d'acide phtalique, des esters bi-fonctionnels d'acide méthacrylique, des esters multifonctionnels d'acide acrylique, le N-méthylène-bis-acrylamide et des monomères aliphatiques portant plusieurs substituants vinyle tels que des diènes, triènes et tétraènes. Des monomères de réticulation peuvent notamment être des divinyl-benzènes, des trivinyl-benzènes, le 1,2,4-trivinylcyclohexène, le 1,5-hexadiène, le 1,5,9-décatriène, le 1,9-décadiène, le 1,5-heptadiène, des di-allyl phthalates, de l'éthylène glycol diméthacrylate, des polyéthylène glycol diméthacrylates, des penta- et tétra-acrylates, des triallyl pentaérythritols, des octaallyl saccharoses, des cycloparaffines, des cyclooléfines et du N-méthylène-bis-acrylamide.

Les agents nacrants et/ou opacifiants de l'invention sont choisis parmi :
i) les esters de polyols à plus de deux atomes de carbone et d'acides gras à chaîne longue, préférentiellement en C₁₀-C₃₀ et encore plus préférentiellement en C₁₆-C₂₂;
ii) les alcanolamides d'acides gras à chaîne longue, préférentiellement en C₁₀-C₃₀ et encore plus préférentiellement en C₁₆-C₂₂, tels que le monoéthanolamide stéarique, le diéthanolamide stéarique, le monoisopropanolamide stéarique ou le stéarate de monoéthanolamide stéarique;
iii) les esters de monoalcools à chaîne longue (C₁₀-C₃₀) et d'acides gras à chaîne longue (C₁₀-C₃₀) comme le cétyl palmitate;
iv) les éthers d'alcools gras à chaîne longue solides à une température inférieure ou égale à 30° C tels que par exemple les dialkyléthers de formule (I):

   R-O-R' (I)

   dans laquelle R et R', identiques ou différents, désignent un radical alkyle, saturé ou insaturé, linéaire ou ramifié, comportant de 10 à 30 atomes de carbone et de préférence de 14 à 24 atomes de carbone, R et R' étant choisis de façon telle que le composé de formule (I) soit solide à une température inférieure ou égale à 30°C. Plus particulièrement, R et R' désignent un radical stéaryle. Ces composés peuvent notamment être préparés selon le procédé décrit dans la demande de brevet DE 41 27 230. Un distéaryléther utilisable dans le cadre de la présente invention, est commercialisé sous la dénomination CUTINA STE par la société HENKEL;
v) les esters à chaîne longue (C₁₀-C₃₀) d'alcanolamides à chaîne longue (C₁₀-C₃₀) tels que le distéarate stéaramide diéthanolamide ou le stéarate stéaramide monoéthanolamide;
vi) les alcools gras monochaînes à plus de 20 atomes de carbone tels que l'alcool béhénique;
vii) les oxydes d'amine à chaîne longue, tels que les alkyl (C₁₀-C₃₀) diméthyl amine oxydes, comme par exemple le stéaryl diméthyl amine oxyde;
viii) les acides benzoïques N,N-dihydrocarbyl(C₁₀-C₃₀, préférentiellement C₁₂-C₂₂) amido et leurs sels et particulièrement l'acide benzoïque N,N-di(C₁₆-C₁₈)amido commercialisé par la société STEFAN COMPANY; et
ix) les alcools ayant de 27 à 48 atomes de carbone et comportant un ou deux groupements éther et/ou thioéther ou sulfoxyde répondant à la formule (II):

   Rₐ-X-[C₂H₃(OH)]-CH₂-Y-R_{b} (II)

   dans laquelle Rₐ et R_{b} désignent indépendamment l'un de l'autre, des groupements linéaires en C₁₂ à C₂₄;
   X désigne un atome d'oxygène, un atome de soufre, un groupement sulfoxyde ou méthylène;
   Y désigne un atome d'oxygène, un atome de soufre, un groupement sulfoxyde ou méthylène;
   la somme du nombre d'atomes de carbone présents dans les groupements Rₐ et R_{b} a une valeur variant de 24 à 44 et de préférence de 28 à 40 inclus;
   lorsque X ou Y désigne sulfoxyde, Y ou X ne désigne pas soufre.
   Les composés de formule (II) utilisés de préférence conformément à l'invention, sont ceux pour lesquels X désigne oxygène, Y désigne méthylène, et Rₐ et R_{b} désignent des radicaux ayant 12 à 22 atomes de carbone, ces composés pouvant être préparés selon le brevet EP 457 688; et
x) les oxydes de titane enrobés ou non, les micas et les mica titanes.

Les agents nacrants et/ou opacifiants sont préférentiellement choisis parmi le distéaryl éther, l'alcool béhénique et le 1-(hexadécyloxy)- 2-octadécanol.
Au moins un agent nacrant et/ou opacifiant doit être présent dans des proportions d'au moins 1,5% en poids, et préférentiellement dans des proportions comprises entre 1,5 et 20%, et encore plus préférentiellement entre 2 et 6% en poids par rapport au poids total de la composition. Ces proportions sont nécessaires afin d'obtenir une composition présentant un aspect nacré et/ou opacifié.

Comme indiqué précédemment, les compositions selon l'invention contiennent au moins un agent tensio-actif détergent choisi parmi les tensio-actifs anioniques, amphotères et non-ioniques ayant des propriétés détergentes, et leurs mélanges.

Parmi les agents tensio-actifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates et les N-acyltaurates.

Le radical alkyle ou acyle de ces différents composés est généralement constitué par une chaîne carbonée comportant de 8 à 30 atomes de carbone.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 30 atomes de carbone.

On peut également utiliser des agents tensio-actifs considérés comme faiblement anioniques tels que les acides alkyl ou alkylaryl éther carboxyliques polyoxyalkylénés ou leurs sels, les acides alkylamido éther carboxyliques polyoxyalkylénés ou leurs sels, les acides d'alkyl D-galactoside uroniques ou leurs sels.

Les agents tensio-actifs non-ioniques sont plus particulièrement choisis parmi les alcools ou les alkylphénols ou les acides gras polyéthoxylés, polypropoxylés ou polyglycérolés, à chaîne grasse comportant 8 à 30 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés avec 2 à 30 moles d'oxyde d'éthylène; les esters d'acide gras de sucrose, les esters d'acides gras du polyéthylèneglycol, les alkylpolyglycosides, les dérivés carbamates ou amides de N-alkyl glucamines, les aldobionamides, les oxydes d'amines tels que les oxydes d'alkylamines ou de N-acylamidopropyl-morpholine.

Les agents tensio-actifs amphotères préférés sont les dérivés d'amines secondaires ou tertiaires aliphatiques, dans lesquels le radical aliphatique est une chaîne linéaire ou ramifiée comportant 8 à 22 atomes de carbone et qui contient au moins un groupe anionique hydrosolubilisant, carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

Parmi les dérivés d'amines, on peut citer les produits vendus sous la dénomination MIRANOL, tels que décrits dans les brevets US-A-2 528 378 et 2 781 354 et classés dans le dictionnaire CTFA, 7ème édition, 1997, sous la dénomination Disodium Cocoamphodiacétate, Disodium Lauroamphodiacétate, Disodium Capryloamphodiacétate, Disodium Caproamphodiacétate, Disodium Cocoamphodipropionate, Disodium Lauroamphodipropionate, Disodium Caproamphodipropionate, Disodium Capryloamphodipropionate, Lauroamphodipropionate acide, Cocoamphodipropionate acide.
Les agents tensio-actifs sont utilisés dans les compositions conformes à l'invention dans des proportions suffisantes pour conférer un caractère détergent à la composition, généralement à raison d'au moins 4% en poids, de préférence entre 5 et 50% en poids par rapport au poids total de la composition et en particulier entre 8 et 35%.

Les compositions, selon l'invention, présentent un pH généralement compris entre 3 et 12, et plus particulièrement entre 4 et 8.

Le milieu cosmétiquement acceptable des compositions est constitué, soit par de l'eau, soit par un ou plusieurs solvants, soit par un mélange d'eau et d'au moins un solvant cosmétiquement acceptable choisi parmi les alcools inférieurs, les alkylèneglycols et les éthers de polyols.

Selon un mode de réalisation préféré de l'invention, les compositions selon la présente invention contiennent des polyorganosiloxanes modifiés ou non, à savoir des huiles de polyorganosiloxanes ou des gommes ou des résines de polyorganosiloxanes, telles quelles ou sous forme de solutions dans des solvants organiques ou encore sous forme d'émulsions ou de microémulsions.

Parmi les polyorganosiloxanes pouvant être utilisés conformément à la présente invention, on peut citer à titre non limitatif :
I. Les silicones volatiles : celles-ci possèdent un point d'ébullition compris entre 60°C et 260°C. Elles sont choisies parmi les silicones cycliques contenant de 3 à 7 atomes de silicium et de préférence 4 à 5. Il s'agit, par exemple, de l'octaméthyl-cyclotétrasiloxane vendu sous le nom de "VOLATILE SILICONE 7207" par UNION CARBIDE ou "SILBIONE 70045 V2" par RHODIA CHIMIE, le décaméthylcyclopentasiloxane vendu sous le nom de "VOLATILE SILICONE 7158" par UNION CARBIDE, "SILBIONE 70045 V5 par RHODIA CHIMIE, ainsi que leurs mélanges. On cite également les cyclocopolymères du type diméthylsiloxane/méthylalkylsiloxane, tel que la "SILICONE VOLATILE FZ3109" vendue par la Société UNION CARBIDE, qui est un cyclocopolymère diméthylsiloxane/méthyloctylsiloxane.
II. Les silicones non volatiles : elles sont constituées principalement par:
   (i) les polyalkylsiloxanes ; Parmi les polyalkylsiloxanes, on peut citer principalement les polydiméthylsiloxanes linéaires à groupements terminaux triméthylsilyle, comme par exemple, et à titre non limitatif, les huiles "SILBIONE" de la série 70047 commercialisées par RHONE POULENC.
   (ii) les polyarylsiloxanes ;
   (iii) les polyalkylarylsiloxanes ; on peut citer les polyméthylphénylsiloxanes, les polydiméthylméthylphénylsiloxanes, les polydiméthyldiphénylsiloxanes linéaires et ramifiés, tels que par exemple l'huile "RHODORSIL 763" de RHODIA CHIMIE;
   (iv) les gommes de silicone ; ce sont des polydiorganosiloxanes de masse moléculaire comprise entre 200 000 et 1 000 000, utilisés seuls ou en mélange dans un solvant choisi parmi les silicones volatiles, les huiles polydiméthylsiloxanes (PDMS), les huiles polyphénylméthylsiloxanes, (PPMS), les isoparaffines, le chlorure de méthylène, le pentane, le dodécane, le tridécane, le tétradécane ou leurs mélanges; elles peuvent posséder des structures du type :
      - polydiméthylsiloxane,
      - poly[(diméthylsiloxane)/(méthylvinylsiloxane)],
      - poly[(diméthylsiloxane)/(diphénylsiloxane)],
      - poly[(diméthylsiloxane))(phénylméthylsiloxane)],
      - poly[(diméthylsiloxane)/(diphénylsiloxane)/(méthylvinylsiloxane)];
   on peut aussi citer, par exemple, à titre non limitatif, les mélanges suivants :
      1) les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401" vendu par la Société DOW CORNING;
      2) les mélanges formés à partir d'une gomme polydiméthylsiloxane avec une silicone cyclique, tel que le produit "SF 1214 SILICONE FLUID" de GENERAL ELECTRIC, qui est une gomme SE 30 de PM 500 000 solubilisée dans la "SF 1202 SILICONE FLUID" (décaméthylcyclopentasiloxane);
      3) les mélanges de deux PDMS de viscosité différente, notamment d'une gomme PDMS et d'une huile PDMS, tels que les produits "SF 1236" et "CF 1241" de la Société GENERAL ELECTRIC;
   (v) les résines de silicone ; de préférence des systèmes siloxaniques réticulés renfermant les unités R₂SiO_{2/2}, RSiO_{3/2} et SiO_{4/2} dans lesquelles R représente un groupement hydrocarboné possédant 1 à 6 atomes de carbone ou un groupement phényle. Parmi ces résines, on peut citer le produit vendu sous la dénomination "DOW CORNING 593";
   (vi) les polyorganosiloxanes organomodifiés ; c'est-à-dire des silicones telles que définies précédemment, comportant dans leur structure générale, un ou plusieurs groupements organofonctionnels directement fixés sur la chaîne siloxanique ou fixés par l'intermédiaire d'un radical hydrocarboné; on cite, par exemple, les silicones comportant :
      a) des groupements polyéthylèneoxy et/ou polypropylèneoxy, comportant éventuellement des groupes alkyle, tels que le produit dénommé diméthicone copolyol vendu par la Société DOW CORNING sous la dénomination "DC 1248", et l'alkyl (C12) méthicone copolyol vendu par la Société DOW CORNING sous la dénomination "Q2 5200";
      b) des groupements (per)fluorés comme les groupements trifluoroalkyle, telles que, par exemple, celles vendues par la Société GENERAL ELECTRIC sous les dénominations "FF.150 Fluorosilicone Fluid";
      c) des groupements hydroxyacylamino, telles que celles décrites dans la demande de brevet européen EP-A-0 342 834 et en particulier la silicone vendue par la Société DOW CORNING sous la dénomination "Q2-8413" ;
      d) des groupements thiols comme dans les silicones "X 2-8360" de DOW CORNING ou les "GP 72A" et "GP 71" de GENESEE ;
      e) des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ou aminoalkyl(C₁-C₄)aminoalkyl(C₁-C₄). On utilise plus particulièrement les silicones dénommées amodiméthicone et triméthylsilylamodiméthicone selon la dénomination CTFA (1997);
      f) des groupements carboxylates, comme les produits décrits dans le brevet européen EP 186 507 de CHISSO CORPORATION;
      g) des groupements hydroxylés, comme les polyorganosiloxanes à fonction hydroxyalkyle, décrits dans la demande de brevet FR-A-2 589 476 ;
      h) des groupements alcoxylés comportant au moins 12 atomes de carbone comme le produit "SILICONE COPOLYMER F 755" de SWS SILICONES;
      i) des groupements acyloxyalkyle comportant au moins 12 atomes de carbone, comme par exemple les polyorganosiloxanes décrits dans la demande de brevet FR-A-2 641 185;
      j) des groupements ammonium quaternaire, comme dans le produit "ABIL K 3270" de la Société GOLDSCHMIDT ;
      k) des groupements amphotères ou bétaïniques, tels que dans le produit vendu par la Société GOLDSCHMIDT sous la dénomination "ABIL B 9950" ;
      l) des groupements bisulfite, tels que dans les produits vendus par la Société GOLDSCHMIDT sous les dénominations "ABIL S 201" et "ABIL S 255";
   (vii) les copolymères blocs ayant un bloc linéaire polysiloxane-polyalkylène comme unité répétitive ; la préparation de tels copolymères blocs mis en oeuvre dans le cadre de la présente invention est décrite dans la demande européenne EP 0 492 657 A1, dont l'enseignement est inclus à titre de référence dans la présente description;
   (viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; notamment ceux choisis plus préférentiellement parmi ceux décrits dans les brevets US 4.963.935, US 4.728.571 et US 4.972.037 et les demandes de brevet EP-A 0 412 704, EP-A-0 412 707, EP-A-0 640 105 et WO 95/00578 dont les enseignement sont totalement inclus dans la présente description à titre de références non limitatives;
   (ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; des exemples de tels polymères, ainsi que leur mode particulier de préparation, sont notamment décrits dans les demandes de brevets EP-A-0 582 152, WO 93/23009 et WO 95/03776 dont les enseignements sont inclus totalement dans la présente description à titre de références non limitatives;
   (x) ou leurs mélanges.
Les polyorganosiloxanes préférés pour être utilisés selon l'invention sont les polydiméthylsiloxanes non volatils aminés ou non.

Les polyorganosiloxanes sont utilisés dans les compositions de l'invention dans des proportions comprises entre 0,01 et 20% en poids et de préférence entre 0,1 et 10% en poids par rapport au poids total de la composition.
Selon un autre mode de réalisation préféré, les compositions de l'invention contiennent en outre au moins un polymère cationique choisi parmi tous ceux déjà connus en soi, notamment ceux décrits dans la demande de brevet EP-A-0 337 354 et dans les demandes de brevets français FR-A-2 270 846, 2 383 660, 2 598 611, 2 470 596 et 2 519 863. Les polymères cationiques utilisés ont généralement une masse moléculaire comprise entre 500 et 5.10⁶ environ, et de préférence comprise entre 10³ et 3.10⁶ environ .
Parmi les polymères cationiques, on peut citer plus particulièrement les protéines (ou hydrolysats de protéines) quaternisées et les polymères du type polyamine, polyaminoamide et polyammonium quaternaire. Ce sont des produits connus.
Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits dénommés dans le dictionnaire CTFA "TRIETHOMIUM HYDROLYZED COLLAGEN ETHOSULFATE";
- les hydrolysats de collagène portant des groupements chlorure de triméthylammonium et de triméthylstéarylammonium, dénommés dans le dictionnaire CTFA "STEARTRIMONIUM HYDROLYZED COLLAGEN" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres le "CROQUAT L", le "CROQUAT M", le "CROQUAT S" et le "CROTEIN Q" vendus par la Société CRODA.
D'autre protéines ou hydrolysats quaternisés sont par exemple ceux vendus par la Société INOLEX, sous la dénomination "LEXEIN QX 3000".
On peut encore citer les protéines végétales quaternisées telles que les protéines de blé, de maïs ou de soja: comme protéines de blé quaternisées, on peut citer celles appelées dans le dictionnaire CTFA "COCODIMONIUM HYDROLYSED WHEAT PROTEIN", "LAURIDIMONIUM HYDROLYSED WHEAT PROTEIN", ou encore "STEARDIMONIUM HYDROLYSED WHEAT PROTEIN".
Les polymères du type polyamine, polyaminoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer:
(1) Les copolymères vinylpyrrolidone-acrylate ou -méthacrylate de dialkylaminoalkyle quaternisés ou non, tels que les polymères décrits en détail dans les brevets français 2 077 143 et 2 393 573.
(2) Les dérivés d'éthers de cellulose comportant des groupements ammonium quaternaires décrits dans le brevet français 1 492 597.
(3) Les dérivés de cellulose cationiques tels que les copolymères de cellulose ou les dérivés de cellulose greffés avec un monomère hydrosoluble d'ammonium quaternaire, et décrits notamment dans le brevet US 4131 576, tels que les hydroxyalkyl celluloses, comme les hydroxyméthyl-, hydroxyéthyl- ou hydroxypropyl celluloses greffées notamment avec un sel de méthacryloyléthyl triméthyl ammonium, de méthacrylamidopropyl triméthyl ammonium ou de diméthyldiallylammonium.
(4) Les polysaccharides et notamment gommes de guar cationiques décrits plus particulièrement dans les brevets US 3 589 578 et 4 031 307.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé, l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés. De tels polymères sont notamment décrits dans les brevets français 2.252.840 et 2.368.508.
(7) Les dérivés de polyaminoamides résultant de la condensation de polyalcoylène polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone, le rapport molaire entre la polyalkylène polyamine et l'acide dicarboxylique étant compris entre 0,8 : 1 et 1,4 : 1, le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(9) Les cyclopolymères de méthyl diallyl amine ou de diméthyl diallyl ammonium, notamment ceux décrits dans le brevet français 2.080.759 et dans son certificat d'addition 2.190.406.
(10) Les polymères de diammonium quaternaire décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) Les polymères de polyammonium quaternaire notamment décrits dans la demande de brevet EP-A-122 324.
(12) Les homopolymères ou copolymères dérivés des acides acrylique ou méthacrylique et comportant des motifs
   CH₂-CHRₐ-CO-O-A₁-NRₑR_{f}, CH₂-CHRₐ-CO-O-A₁-N⁺R_{b}R_{c}R_{d}, X⁻ et/ou CH₂-CHRₐ-CO-NH-A₁-N⁺R_{b}R_{c}R_{d}, X⁻
   dans lesquels les groupements Rₐ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone, les groupements R_{b}, R_{c}, R_{d}, identiques ou différents, désignent indépendamment un groupe alcoyle de 1 à 18 atomes de carbone ou un radical benzyle, les groupements Rₑ et R_{f} représentent un atome d'hydrogène ou un groupement alcoyle de 1 à 6 atomes de carbone, X⁻ désigne un anion, par exemple méthosulfate ou halogénure, tel que chlorure ou bromure,
(13) Les polymères quaternaires de vinylpyrrolidone et de vinylimidazole tels que par exemple les produits commercialisés sous les dénominations "LUVIQUAT FC 905", "LUVIQUAT FC 550" et "LUVIQUAT FC 370" par la société BASF.
(14) Les polyamines comme le "POLYQUART H" vendu par HENKEL, référencé sous le nom de « POLYETHYLENEGLYCOL TALLOW POLYAMINE » dans le dictionnaire CTFA.
(15) Les polymères réticulés de sel de méthacryloyloxyéthyl triméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléhnique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale.
D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.
Parmi tous les polymères cationiques susceptibles d'être utilisés dans le cadre de la présente invention, on préfère les dérivés d'éther de cellulose comportant des groupements ammonium quaternaires, les polysaccharides et notamment gomme de guar cationiques et les cyclopolymères de méthyl diallyl amine ou de diméthyl dialyl ammonium.

Les polymères cationiques sont utilisés dans les compositions de l'invention dans des proportions comprises entre 0,001 et 20% en poids et de préférence entre 0,05 et 5% en poids par rapport au poids total de la composition.

Selon un mode de réalisation particulièrement préféré, les compositions de l'invention contiennent en outre au moins une silicone et au moins un polymère cationique.

Les compositions selon l'invention peuvent également contenir en outre au moins un adjuvant choisi parmi les adjuvants habituellement utilisés en cosmétique, tels que les parfums, les conservateurs, les séquestrants, les humectants, les sucres, les huiles végétales, minérales, animales ou de synthèse, les polymères amphotères, les tensio-actifs cationiques, le menthol, les dérivés de nicotinate, les agents anti-chute des cheveux, les agents antipelliculaires, les stabilisateurs de mousse, les agents propulseurs, les filtres, les colorants, les céramides, les vitamines ou provitamines et les agents acidifiants ou alcalinisants.

Dans une forme de réalisation préférée de l'invention, les compositions selon l'invention sont utilisées comme shampooings pour le lavage et le traitement des cheveux.

Le procédé de lavage des matières kératiniques consiste à appliquer une composition telle que définie ci-dessus sur des matières kératiniques humides ou sèches dans des quantités efficaces pour les laver, cette application étant suivie d'un rinçage après un temps de pose facultatif.

Les exemples qui suivent sont destinés à illustrer l'invention.

| **EXEMPLE I : SHAMPOOING** | |
|---|---|
| Cocoyl bétaïne en solution aqueuse à 30% | 8 g |
| Chlorure d'hydroxypropyl guar triméthyl ammonium vendu par la société MEYHALL sous la dénomination "JAGUARC13S" | 0,05 g |
| Polydiméthylsiloxane de viscosité de 0,3m².s⁻¹ vendu par la société DOW CORNING sous la dénomination "DC 200 FLUID 300.000" | 2,7 g |
| Mélange 1-(hexadécyloxy)-2-octadécanol / alcool cétylique | 2,5 g |
| Monoisopropanolamide d'acides de coprah | 0,5 g |
| Lauryl éther sulfate de sodium (2,2 OE) à 70% de M.A. | 22 g |
| Terpolymère d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A. vendu par la société National Starch sous la dénomination "STRUCTURE ® PLUS" | 1 g |
| Parfum, conservateurs qs | |
| Eau déminéralisée stérilisée qsp | 100 g |

On ajuste le pH à 7,5 par de l'acide citrique ou par de la soude.

Après utilisation de ce shampooing, on constate sur cheveux mouillés de la douceur et sur cheveux séchés de la douceur, de la brillance, un toucher lisse, de la souplesse et une bonne malléabilité.

| **EXEMPLE II : SHAMPOOING** | |
|---|---|
| Propylène glycol | 0,1 g |
| Cocoyl bétaïne en solution aqueuse à 30% | 10 g |
| Chlorure d'hydroxypropyl guar trimethyl ammonium vendu par la société MEYHALL sous la dénomination "JAGUAR C13S" | 0,1 g |
| Mélange 1-(hexadécyloxy)-2-octadécanol / alcool cétylique | 2,5 g |
| monoisopropanolamide d'acides de coprah | 0,6 g |
| Poly diméthylsiloxane à groupements aminoéthyl iminopropyl en émulsion cationique à 35% dans l'eau vendu par la société DOW CORNING sous la dénomination "DC 939" | 7 g |
| Lauryl éther sulfate de sodium (2,2 OE) à environ 70% de M.A. | 22 g |
| Terpolymère d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A. vendu par la société National Starch sous la dénomination "STRUCTURE® PLUS" | 1 g |
| conservateurs qs | |
| Eau déminéralisée stérilisée qsp | 100 g |

On ajuste le pH à 5 par de l'acide citrique ou par de la soude.

Après utilisation de ce shampooing, on constate sur cheveux mouillés de la douceur et sur cheveux séchés de la douceur, de la brillance, un toucher lisse, de la souplesse et une bonne malléabilité.

| **EXEMPLE III : SHAMPOOING** | |
|---|---|
| N-cocoyl amidoéthyl, N-éthoxycarboxyméthyl glycinate de sodium à 38% M.A. vendu sous la dénomination "MIRANOL C2M CONC" par la société RHODIA CHIMIE | 1,5 g |
| Cocoyl bétaïne en solution aqueuse à 30% | 6 g |
| Monoisopropanolamide d'acides de coprah | 1 g |
| Alcool laurique oxyéthyléné (2,5 OE) | 0,75 g |
| Lauryl éther sulfate de sodium (2,2 OE) à 70% de M.A. | 16,75 g |
| Distéaryl éther | 1,5 g |
| Terpolymère d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A. vendu par la société National Starch sous la dénomination "STRUCTURE ® PLUS" | 2,5 g |
| Mélange d'alcools linéaires (C18/C20/C22) | 1,5 g |
| Parfum, conservateurs qs | |
| Eau déminéralisée stérilisée qsp | 100 g |

On ajuste le pH à 6,5 par de l'acide citrique ou par de la soude.

Après utilisation de ce shampooing, on constate sur cheveux mouillés de la douceur et sur cheveux séchés de la douceur, de la brillance, un toucher lisse, de la souplesse et une bonne malléabilité.

| **EXEMPLE IV : SHAMPOOING** | |
|---|---|
| Chlorure de sodium | 0,3 g |
| Vitamine B3 ou PP : amide nicotinique qs | |
| N-cocoyl amidoéthyl, N-éthoxycarboxyméthyl glycinate de sodium (38 %) | 1,5 g |
| Vitamine B6 : chlorhydrate de pyridoxine qs | |
| Chlorure d'hydroxypropyl guar triméthyl ammonium vendu sous le dénomination "JAGUAR C 13S" par la société MEYHALL | 0,04 g |
| Polydiméthylsiloxane de viscosité 0,3m².s⁻¹ vendu sous le dénomination DC 200/300.000 par la société DOW CORNING | 1,8 g |
| Alcool laurique oxyéthyléné (2,5 OE) | 0,75 g |
| Extrait de fruits en solution aqueuse qs | |
| Monoisopropanolamide d'acides de coprah | 2 g |
| Cocoyl amidopropyl betaïne en solution aqueuse à 38% | 2,7 g |
| Lauryl éther sulfate de sodium (2,2 OE) à 70% | 17 g |
| PYRUS MALUS (INCI) qs | |
| Distéaryl éther | 1,5 g |
| Terpolymère d'acrylates, d'amino(méth)acrylates et d'itaconate d'alkyle en C₁₀-C₃₀ polyoxyéthyléné à 20 moles d'oxyde d'éthylène en dispersion aqueuse à 20% M.A. vendu sous la dénomination "STRUCTURE® PLUS" par la Société National Starch | 1 g |
| mélange d'alcools linéaires(C18/C20/C22) | 1,5 g |
| Parfum, conservateurs qs | |
| Eau déminéralisée stérilisée qsp | 100 g |

On ajuste le pH à 7,5 par de l'acide citrique ou de l'hydroxyde de sodium. Après utilisation de ce shampooing, on constate sur cheveux mouillés de la douceur et sur cheveux séchés de la douceur, de la brillance, un toucher lisse, de la souplesse et une bonne malléabilité.

## Revendications

1. Composition de lavage des matières kératiniques, comprenant dans un milieu cosmétiquement acceptable:
1) au moins un agent tensio-actif détergent anionique, amphotère ou non-ionique;
2) au moins 1,5% en poids par rapport au poids total de la composition d'au moins un agent nacrant et/ou opacifiant choisi parmi :
i) les esters de polyols à plus de deux atomes de carbone et d'acides gras à chaîne longue;
ii) les alcanolamides d'acides gras à chaîne longue;
iii) les esters de monoalcools à chaîne longue et d'acides gras à chaîne longue;
iv) les éthers d'alcools gras à chaîne longue;
v) les esters à chaîne longue d'alcanolamides à chaîne longue;
vi) les alcools gras monochaînes à plus de 20 atomes de carbone;
vii) les oxydes d'amines à chaîne longue;
viii) les acides benzoïques N,N-dihydrocarbyl(C₁₀-C₃₀, préférentiellement C₁₂-C₂₂) amido et leurs sels;
ix) les alcools ayant de 27 à 48 atomes de carbone et comportant un ou deux groupements éther et/ou thioéther ou sulfoxyde; et
x) les oxydes de titane et les micas;
3) et au moins un terpolymère acrylique constitué:
- de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
- de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di-(C₁-C₄)alkylamino (C₁-C₄) alkyl (méth)acrylamide;
- de 0,1 à 30% en poids, préférentiellement de 0,1 à 10% en poids d'un monomère (c) choisi parmi :
un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
un monomère tensioactif insaturé éthylénique copolymérisable obtenu par condensation d'un tensioactif non-ionique avec un acide carboxylique insaturé α,β-éthylénique ou son anhydride;
un monomère tensioactif choisi parmi les produits de réaction de type urée d'un monoisocyanate insaturé monoéthylénique avec un tensioactif non-ionique présentant une fonction amine;
un éther de (méth) allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne l'éthylèneoxy, n est égal à zéro ou désigne un nombre entier inférieur ou égal à 200 et préférentiellement inférieur à 100, m et p désignent zéro ou un nombre entier inférieur à n et R₂ est un groupe hydrophobe d'au moins 8 atomes de carbone et préférentiellement en C₈-C₃₀; et
un monomère non-ionique de type uréthane produit par réaction d'un tensioactif non ionique monohydrique avec un isocyanate insaturé monoéthylénique;
les pourcentages en poids de monomères étant basés sur le poids total des monomères constituant le terpolymère.

2. Composition selon la revendication 1, caractérisée en ce que le terpolymère est présent à raison de 0,01 à 20% en poids de matière active, de préférence 0,1 à 10 % en poids par rapport au poids total de la composition.

3. Composition selon la revendication 1 ou 2, caractérisée en ce que le monomère (a) est choisi parmi les acrylates d'alkyle en C₂-C₆, et est préférentiellement l'acrylate d'éthyle.

4. Composition selon l'une quelconque des revendications 1 à 3, caractérisée en ce que le monomère (b) est choisi parmi le méthacrylate de N,N-diméthylaminoéthyle, l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyl-méthacrylamide, le N,N-diéthylaminopropyl-acrylamide et le N,N-diéthylaminopropyl-méthacrylamide, et est préférentiellement le méthacrylate de N,N-diméthylaminoéthyle.

5. Composition selon l'une quelconque des revendications 1 à 4, caractérisée en ce que le monomère (c) est un monomère tensioactif insaturé éthylénique copolymérisable obtenu par condensation d'un tensioactif non-ionique avec l'acide itaconique.

6. Composition selon l'une quelconque des revendications 1 à 5, caractérisée en ce que le terpolymère acrylique est constitué d'acrylates, d'amino(méth)acrylates et d'itaconate d'alkyle en C₁₀-C₃₀ polyoxyéthyléné à 20 moles d'oxyde d'éthylène.

7. Composition selon l'une quelconque des revendications 1 à 6, caractérisée en ce que le terpolymère acrylique contient en outre un monomère de réticulation.

8. Composition selon l'une quelconque des revendications 1 à 7, caractérisée en ce que l'agent nacrant et/ou opacifiant est choisi parmi:
i) les esters de polyols à plus de deux atomes de carbone et d'acides gras à chaîne longue en C₁₀-C_{30,} préférentiellement en C₁₆-C₂₂;
ii) les alcanolamides d'acides gras à chaîne longue en C₁₀-C₃₀ , préférentiellement en C₁₆-C₂₂, tels que le monoéthanolamide stéarique, le diéthanolamide stéarique, le monoisopropanolamide stéarique ou le stéarate de monoéthanolamide stéarique;
iii) les esters de monoalcools à chaîne longue (C₁₀-C₃₀) et d'acides gras à chaîne longue (C₁₀-C₃₀) comme le cétyl palmitate;
iv) les éthers d'alcools gras à chaîne longue solides à une température inférieure ou égale à 30° C tels que les dialkyléthers de formule (I):
R-O-R' (I)
dans laquelle R et R', identiques ou différents, désignent un radical alkyle, saturé ou insaturé, linéaire ou ramifié, comportant de 10 à 30 atomes de carbone et de préférence de 14 à 24 atomes de carbone, R et R' étant choisis de façon telle que le composé de formule (I) soit solide à une température inférieure ou égale à 30°C;
v) les esters à chaîne longue (C₁₀-C₃₀) d'alcanolamides à chaîne longue (C₁₀-C₃₀) tels que le distéarate stéaramide diéthanolamide ou le stéarate stéaramide monoéthanolamide;
vi) les alcools gras monochaînes à plus de 20 atomes de carbone tels que l'alcool béhénique;
vii) les oxydes d'amine à chaîne longue, tels que les alkyl (C₁₀-C₃₀) diméthyl amine oxydes, comme par exemple le stéaryl diméthyl amine oxyde;
viii) les acides benzoïques N,N-dihydrocarbyl(C₁₀-C₃₀, préférentiellement C₁₂-C₂₂) amido et leurs sels et particulièrement l'acide benzoïque N,N-di(C₁₆-C₁₈)amido;
ix) les alcools ayant de 27 à 48 atomes de carbone et comportant un ou deux groupements éther et/ou thioéther ou sulfoxyde répondant à la formule (II):
Rₐ-X-[C₂H₃(OH)]-CH₂-Y-R_{b} (II)
dans laquelle Rₐ et R_{b} désignent indépendamment l'un de l'autre, des groupements linéaires en C₁₂ à C₂₄;
X désigne un atome d'oxygène, un atome de soufre, un groupement sulfoxyde ou méthylène;
Y désigne un atome d'oxygène, un atome de soufre, un groupement sulfoxyde ou méthylène;
la somme du nombre d'atomes de carbone présents dans les groupements Rₐ et R_{b} a une valeur variant de 24 à 44 et de préférence de 28 à 40 inclus; lorsque X ou Y désigne sulfoxyde, Y ou X ne désigne pas soufre; et
x) les oxydes de titane enrobés ou non, les micas et les mica titanes.

9. Composition selon la revendication 8, caractérisée par le fait que l'agent nacrant et/ou opacifiant est choisi parmi le distéaryl éther, l'alcool béhénique et le 1-(hexadécyloxy)- 2-octadécanol.

10. Composition selon l'une quelconque des revendications 1 à 9, caractérisée par le fait que l'agent nacrant et/ou opacifiant est présent dans des proportions d'au moins 1,5%, et préférentiellement dans des proportions comprises entre 1,5 et 20%, en poids par rapport au poids total de la composition et encore plus préférentiellement entre 2 et 6%.

11. Composition selon l'une quelconque des revendications 1 à 10, caractérisée par le fait que les agents tensio-actifs anioniques sont choisis parmi les sels alcalins, les sels de magnésium, les sels d'ammonium, les sels d'amines ou les sels d'aminoalcools des composés suivants : les alkylsulfates, alkyléthersulfates, alkylamidoéthersulfates, alkylarylpolyéthersulfates, monoglycérides sulfates; les alkylsulfonates, alkylamides sulfonates, alkylaryl-sulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates, N-acyltaurates;le radical alkyle ou acyle de ces différents composés étant constitué par une chaîne carbonée comportant de 8 à 30 atomes de carbone; les sels d'acides gras des acides oléique, ricinoléique, palmitique, stéarique; les acides d'huile de coprah ou d'huile de coprah hydrogénée; les acyl lactylates, dont le radical acyle comporte de 8 à 30 atomes de carbone; les acides d'alkyl D-galactoside uroniques et leurs sels, les acides alkyl ou alkylaryl éther carboxyliques polyoxyalkylénés ou leurs sels, les acides alkylamido éther carboxyliques polyoxyalkylénés ou leurs sels.

12. Composition selon l'une quelconque des revendications 1 à 10, caractérisée par le fait que les agents tensio-actifs non-ioniques sont choisis parmi les alcools ou les alkylphénols ou les acides gras polyéthoxylés, polyoxypropylénés ou polyglycérolés, à chaîne grasse comportant 8 à 30 atomes de carbone, le nombre de groupements oxyde d'éthylène, oxyde de propylène étant compris entre 2 et 50 et le nombre de groupements glycérol étant compris entre 2 et 30; les copolymères d'oxyde d'éthylène et de propylène; les condensats d'oxyde d'éthylène et de propylène sur des alcools gras; les amides gras polyéthoxylés; les amides gras polyglycérolés; les amines grasses polyéthoxylées; les esters d'acides gras du sorbitan oxyéthylénés; les esters d'acides gras de sucrose ou du polyéthylèneglycol; les alkylpolyglycosides; les dérivés amides ou carbamates de N-alkylglucamines, les aldobionamides et les oxydes d'amines.

13. Composition selon l'une quelconque des revendications 1 à 10, caractérisée par le fait que les agents tensio-actifs amphotères sont choisis parmi les dérivés d'amines secondaires ou tertiaires aliphatiques, dans lesquels le radical aliphatique est une chaîne linéaire ou ramifiée comportant de 8 à 22 atomes de carbone et qui contient au moins un groupe anionique hydrosolubilisant, carboxylate, sulfonate, sulfate, phosphate, phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

14. Composition selon l'une quelconque des revendications 1 à 13, caractérisée en ce que l'agent tensio-actif détergent est présent à raison d'au moins 4% en poids, de préférence de 5 à 50% en poids, et encore plus préférentiellement de 8 à 35% en poids par rapport au poids total de la composition.

15. Composition selon l'une quelconque des revendications 1 à 14, caractérisée en ce qu'elle présente un pH compris entre 3 et 12, et plus particulièrement entre 4 et 8.

16. Composition selon l'une quelconque des revendications 1 à 15, caractérisée par le fait que le milieu cosmétiquement acceptable est constitué par de l'eau, par un ou plusieurs solvants ou par un mélange d'eau et d'au moins un solvant choisi parmi les alcools inférieurs, les alkylèneglycols et les éthers de polyol.

17. Composition selon l'une quelconque des revendications 1 à 16, caractérisée par le fait qu'elle contient en outre au moins un polyorganosiloxane choisi parmi les silicones volatiles et les silicones non-volatiles comprenant:
(i) les polyalkylsiloxanes ;
(ii) les polyarylsiloxanes ;
(iii) les polyalkylarylsiloxanes ;
(iv) les gommes de silicone ;
(v) les résines de silicone ;
(vi) les polyorganosiloxanes organomodifiés ;
(vii) les copolymères blocs ayant un bloc linéaire polysiloxane-polyalkylène comme unité répétitive ;
(viii) les polymères siliconés greffés, à squelette organique non siliconé;
(ix) les polymères siliconés greffés, à squelette polysiloxanique greffé par des monomères organiques non siliconés;
(x) et leurs mélanges.

18. Composition selon la revendication 17, caractérisée par le fait qu'elle contient un polyorganosiloxane dans des proportions comprises entre 0,01 et 20% en poids et de préférence entre 0,1 et 10% en poids par rapport au poids total de la composition.

19. Composition selon l'une quelconque des revendications 1 à 18, caractérisée par le fait qu'elle contient en outre au moins un polymère cationique choisi parmi :
- les protéines ou hydrolysats de protéines, en particulier les hydrolysats de collagène portant des groupements triéthylammonium, les hydrolysats de collagène portant des groupements chlorure de triméthylammonium et de triméthylstéarylammonium, les hydrolysats de protéines portant sur la chaîne polypeptidique des groupements ammonium quaternaire comportant au moins un radical alkyle ayant de 1 à 18 atomes de carbone, les protéines végétales quaternisées telles que les protéines de blé, de maïs ou de soja;
- les polymères du type polyamine, polyaminoamide, polyammonium quaternaire, en particulier :
i) les copolymères vinylpyrrolidone-acrylate ou méthacrylate de dialkylaminoalkyle quaternisés ou non,
ii) les dérivés d'éthers de cellulose comportant des groupements ammonium quaternaires,
iii) les dérivés de cellulose cationiques tels que les copolymères de cellulose ou les dérivés de cellulose greffés avec un monomère hydrosoluble d'ammonium quaternaire,
iv) les polysaccharides et notamment gommes de guar cationiques,
v) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères,
vi) les polyaminoamides solubles dans l'eau préparés en particulier par polycondensation d'un composé acide avec une polyamine,
vii) les dérivés de polyaminoamides résultant de la condensation de polyalcoylène polyamines avec des acides polycarboxyliques suivie d'une alcoylation par des agents bifonctionnels,
viii) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,
ix) les cyclopolymères de méthyl diallyl amine ou de diméthyl diallyl ammonium,
x) les polymères de diammonium quaternaire,
xi) les polymères de polyammonium quaternaire,
xii) les homopolymères ou copolymères dérivés des acides acrylique ou méthacrylique et comportant des motifs
CH₂-CHRₐ-CO-O-A₁-NRₑR_{f}, CH₂-CHRₐ-CO-O-A₁-N⁺R_{b}R_{c}R_{d}, X⁻ et/ou CH₂-CHRₐ-CO-NH-A₁-N⁺R_{b}R_{c}R_{d}, X⁻
dans lesquels les groupements Rₐ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone, les groupements R_{b}, R_{c}, R_{d}, identiques ou différents, désignent indépendamment un groupe alcoyle de 1 à 18 atomes de carbone ou un radical benzyle, les groupements Rₑ et R_{f} représentent un atome d'hydrogène ou un groupement alcoyle de 1 à 6 atomes de carbone, X⁻ désigne un anion, par exemple méthosulfate ou halogénure, tel que chlorure ou bromure,
xiii) les polymères quaternaires de vinylpyrrolidone et de vinylimidazole,
xiv) les polyamines référencées sous le nom de "POLYETHYLENEGLYCOL TALLOW POLYAMINE" dans le dictionnaire CTFA,
xv) les polymères réticulés de sel de méthacryloyloxyéthyl triméthylammonium,
- les polyalkylèneimines, les polymères contenant des motifs vinylpyridine ou vinylpyridinium, les condensats de polyamines et d'épichlorhydrine, les polyuréylènes quaternaires et les dérivés de la chitine.

20. Composition selon la revendication 19, caractérisée par le fait qu'elle contient un polymère cationique dans des proportions comprises entre 0,001 et 20% en poids et de préférence entre 0,05 et 5% en poids par rapport au poids total de la composition.

21. Composition selon l'une quelconque des revendications 1 à 20, caractérisée par le fait qu'elle contient en outre au moins un adjuvant cosmétiquement acceptable choisi parmi les parfums, les conservateurs, les séquestrants, les humectants, les sucres, les huiles végétales, minérales, animales ou de synthèse, les polymères amphotères, les tensio-actifs cationiques, le menthol, les dérivés de nicotinate, les agents antichute des cheveux, les agents antipelliculaires, les stabilisateurs de mousse, les agents propulseurs, les filtres, les colorants, les céramides, les vitamines ou provitamines et les agents acidifiants ou alcalinisants.

22. Utilisation comme shampooing de la composition telle que définie dans l'une quelconque des revendications 1 à 21.

23. Procédé de lavage des matières kératiniques, caractérisé par le fait que l'on applique sur ces matières au moins une composition telle que définie dans l'une quelconque des revendications 1 à 21, et après un temps de pose facultatif, on les rince à l'eau.
